(19) Europäisches Patentamt
European Patent Office
Office européen des brevets

(11) Veröffentlichungsnummer: **0 379 915 A1**

(12) **EUROPÄISCHE PATENTANMELDUNG**

(21) Anmeldenummer: **90100701.3**

(22) Anmeldetag: **13.01.90**

(51) Int. Cl.⁵: **C07C 255/54, C07C 255/59, C07C 253/00, A01N 37/34, A01N 37/38**

(30) Priorität: **26.01.89 DE 3902288**

(43) Veröffentlichungstag der Anmeldung: **01.08.90 Patentblatt 90/31**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Busse, Ulrich, Dr.**
**Am Bandenfeld 106**
**D-5657 Haan 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Lürssen, Klaus, Dr.**
**August-Kierspel-Strasse 145**
**D-5060 Bergisch Gladbach 2(DE)**
Erfinder: **Strang, Harry, Dr.**
**Heiderweg 53**
**D-4000 Düsseldorf 31(DE)**

(54) **Substituierte Phenoxybenzonitril-Derivate, Verfahren zu ihrer Herstellung und ihre Verwendung als Herbizide und Pflanzenwuchsregulatoren.**

(57) Die Erfindung betrifft neue substituierte Phenoxybenzonitril-Derivate der Formel (I),

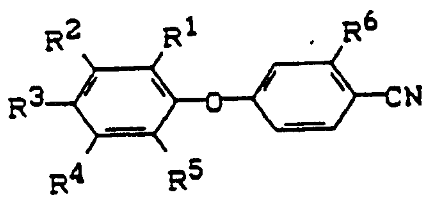

(I)

in welcher
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy oder Trifluormethylsulfonyl steht,
$R^4$ für Wasserstoff oder Halogen steht,
$R^5$ für Wasserstoff oder Halogen steht und
$R^6$ für die Gruppierungen

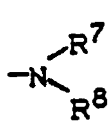

EP 0 379 915 A1

oder

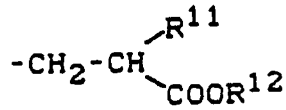

steht,

mehrere Verfahren zu ihrer Herstellung und ihre Verwendung als Herbizide und Pflanzenwuchsregulatoren.

## Substituierte Phenoxybenzonitril-Derivate, Verfahren zu ihrer Herstellung und ihre Verwendung als Herbizide und Pflanzenwuchsregulatoren

Die Erfindung betrifft neue substituierte Phenoxybenzonitril-Derivate, Verfahren zu ihrer Herstellung sowie ihre Verwendung als Herbizide und Pflanzenwuchsregulatoren.

Es ist bereits bekannt, daß bestimmte Phenoxybenzoesäure-Derivate, wie z. B. 3-(2,4-Dichlor-phenoxy)-6-nitrobenzoesäure-methylester (Bifenox) herbizid wirksam sind (vgl. US-P 3 652 645 und US-P 3 776 715). Die Wirkung dieser bekannten Verbindungen ist jedoch nicht immer zufriedenstellend.

Es wurden nun neue substituierte Phenoxybenzonitril-Derivate der allgemeinen Formel (I)

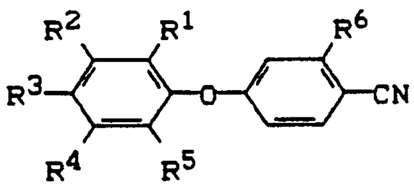

(I)

in welcher
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy oder Trifluormethylsulfonyl steht,
$R^4$ für Wasserstoff oder Halogen steht,
$R^5$ für Wasserstoff oder Halogen steht und
$R^6$ für die Gruppierungen

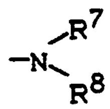

oder

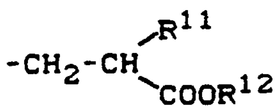

steht, worin
$R^7$ und $R^8$ gleich oder verschieden sind und unabhängig voneinander jeweils für Wasserstoff, Alkoxycarbonylethyl, die Gruppierung $-CO-R^9$ oder die Gruppierung $-SO_2-R^{10}$ stehen, wobei
$R^9$ für jeweils gegebenenfalls substituiertes Alkyl, Alkenyl, Alkinyl, Cycloalkyl, Cycloalkylalkyl, Benzyl, Phenyl, Naphthyl, Pyridyl, Furyl, Thienyl, Alkoxy, Phenoxy, Alkylthio, Phenylthio, Alkylamino, Dialkylamino, Cycloalkylamino oder Phenylamino steht und
$R^{10}$ für jeweils gegebenenfalls substituiertes Alkyl, Phenyl, Naphthyl, Pyridyl oder Thienyl steht,
$R^{11}$ für Wasserstoff oder Halogen steht und
$R^{12}$ für Alkyl steht,
gefunden.

Weiter wurde gefunden, daß man die neuen substituierten Phenoxybenzonitril-Derivate der allgemeinen Formel (I) erhält, wenn man

(a) für den Fall, daß in Formel (I) $R^6$ für Amino steht und $R^1$, $R^2$, $R^3$, $R^4$ und $R^5$ die oben angegebenen Bedeutungen haben,
Halogen-benzol-Derivate der allgemeinen Formel (II)

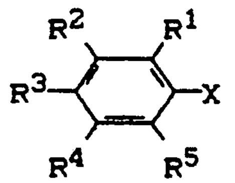

(II)

in welcher
$R^1$, $R^2$, $R^3$, $R^4$ und $R^5$ die oben angegebenen Bedeutungen haben und
X für Halogen steht,
mit 2-Amino-4-hydroxy-benzonitril der Formel (III)

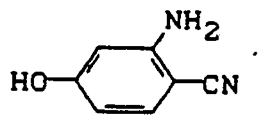

(III)

gegebenenfalls in Gegenwart eines Säureakzeptors und gegebenenfalls in Gegenwart eines Verdünnungsmittels umsetzt, oder wenn man

(b) für den Fall, daß in Formel (I) $R^6$ für die Gruppierung

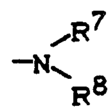

steht, worin $R^7$ und/oder $R^8$ für die Gruppierung $-CO-R^9$ oder die Gruppierung $-SO_2-R^{10}$ stehen, und $R^1$, $R^2$, $R^3$, $R^4$, $R^5$, $R^9$ und $R^{10}$ die oben angegebene Bedeutung haben,
Verbindungen der allgemeinen Formel (I), in welcher $R^6$ für Amino steht und $R^1$, $R^2$, $R^3$, $R^4$ und $R^5$ die oben angegebenen Bedeutungen haben,
mit Verbindungen der allgemeinen Formel (IV)
$X^1-CO-R^9$ (IV)
in welcher
$R^9$ die oben angegebene Bedeutung hat und
$X^1$ für Halogen steht,
oder mit Verbindungen der allgemeinen Formel (V)
$X^2-SO_2-R^{10}$ (V)
in welcher
$R^{10}$ die oben angegebene Bedeutung hat und
$X^2$ für Halogen steht,
gegebenenfalls in Gegenwart eines Säureakzeptors und gegebenenfalls in Gegenwart eines Verdünnungsmittels umsetzt, oder wenn man

(c) für den Fall, daß in Formel (I) $R^6$ für die Guppierung

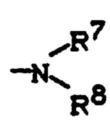

steht, worin $R^7$ und/oder $R^8$ für Alkoxycarbonylethyl stehen, und $R^1$, $R^2$, $R^3$, $R^4$ und $R^5$ die oben angegebenen Bedeutungen haben,
Verbindungen der allgemeinen Formel (I), in welcher $R^6$ für Amino steht und $R^1$, $R^2$, $R^3$, $R^4$ und $R^5$ die oben angegebenen Bedeutungen haben,
mit Acrylsäureestern der Formel (VI)
$CH_2=CH-COOR^{12}$ (VI)

in welcher
$R^{12}$ für Alkyl steht,
gegebenenfalls in Gegenwart eines basischen Katalysators und gegebenenfalls in Gegenwart eines Verdünnungsmittels umsetzt, oder wenn man

(d) für den Fall, daß in Formel (I) $R^6$ für die Gruppierung

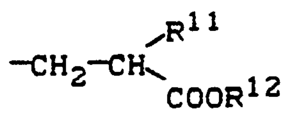

steht, worin $R^{11}$ für Halogen steht und $R^{12}$ für Alkyl steht sowie $R^1$, $R^2$, $R^3$, $R^4$ und $R^5$ die oben angegebenen Bedeutungen haben,
Verbindungen der allgemeinen Formel (I), in welcher $R^6$ für Amino steht und $R^1$, $R^2$, $R^3$, $R^4$ und $R^5$ die oben angegebenen Bedeutungen haben,
mit Natriumnitrit oder Kaliumnitrit und mit einem Hydrogenhalogenid ($HX^3$) in Gegenwart von Wasser und gegebenenfalls in Gegenwart eines organischen Lösungsmittels umsetzt und die hierbei gebildeten Diazoniumsalze der allgemeinen Formel (VII)

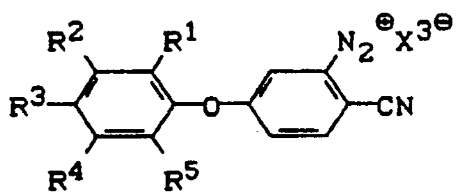
(VII)

in welcher
$R^1$, $R^2$, $R^3$, $R^4$ und $R^5$ die oben angegebenen Bedeutungen haben und
$X^3$ für Halogen steht,
mit Acrylsäureestern der Formel (VI)
$CH_2=CH\text{-}COOR^{12}$ (VI)
in welcher
$R^{12}$ für Alkyl steht,
in Gegenwart von Hydrogenhalogeniden ($HX^3$), gegebenenfalls in Gegenwart von Katalysatoren und gegebenenfalls in Gegenwart von Wasser und dem gegebenenfalls bei der Erzeugung der Verbindungen der Formel (VII) verwendeten organischen Lösungsmittel umsetzt, oder wenn man

(e) für den Fall, daß in Formel (I) $R^6$ für die Gruppierung

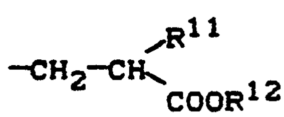

steht, worin $R^{11}$ für Wasserstoff steht und $R^{12}$ für Alkyl steht sowie $R^1$, $R^2$, $R^3$, $R^4$ und $R^5$ die oben angegebenen Bedeutungen haben,
Verbindungen der allgemeinen Formel (I), in welcher
$R^6$ für die Gruppierung

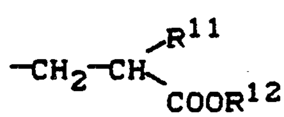

steht, worin $R^{11}$ für Halogen steht und $R^{12}$ für Alkyl steht sowie $R^1$, $R^2$, $R^3$, $R^4$ und $R^5$ die oben angegebenen Bedeutungen haben,

mit einem Dehalogenierungsmittel, gegebenenfalls in Gegenwart eines Katalysators und gegebenenfalls in Gegenwart eines Verdünnungsmittels, umsetzt.

Schließlich wurde gefunden, daß die neuen substituierten Phenoxybenzonitril-Derivate der Formel (I) hervorragende herbizide bzw. pflanzenwuchsregulierende Eigenschaften aufweisen.

Überraschenderweise sind die erfindungsgemäßen substituierten Phenoxybenzonitril-Derivate der Formel (I) gegen Unkräuter wesentlich stärker wirksam als 3-(2,4-Dichlor-phenoxy)-6-nitro-benzoe-säure-methylester, welches ein strukturell ähnlicher vorbekannter Wirkstoff gleicher Wirkungsrichtung ist.

Die Erfindung betrifft vorzugsweise Verbindungen der Formel (I), in welcher

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Trifluormethyl, Trifluormethoxy oder Trifluormethylsulfonyl steht,

$R^4$ für Wasserstoff, Fluor oder Chlor steht,

$R^5$ für Wasserstoff, Fluor oder Chlor steht und

$R^6$ für die Gruppierungen

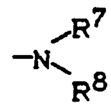

oder

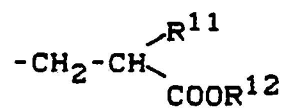

steht, worin

$R^7$ und $R^8$ gleich oder verschieden sind und unabhängig voneinander jeweils für Wasserstoff, $C_1$-$C_6$-Alkoxy-carbonyl-ethyl, die Gruppierung -CO-$R^9$ oder die Gruppierung -$SO_2$-$R^{19}$ stehen, wobei

$R^9$ für gegebenenfalls durch Fluor, Chlor, Brom, $C_1$-$C_4$-Alkoxy und/oder $C_1$-$C_4$-Alkoxy-carbonyl substituiertes $C_1$-$C_6$-Alkyl, für gegebenenfalls durch Fluor, Chlor und/oder Brom substituiertes $C_2$-$C_4$-Alkenyl, für $C_2$-$C_4$-Alkinyl, für gegebenenfalls durch Fluor, Chlor, Brom, $C_1$-$C_4$Alkyl und/oder $C_1$-$C_4$-Alkoxy-carbonyl substi tuiertes $C_3$-$C_6$-Cycloalkyl, für gegebenenfalls durch Fluor, Chlor, Brom und/oder $C_1$-$C_4$-Alkyl substituiertes $C_3$-$C_6$-Cycloalkyl-$C_1$-$C_2$-alkyl, für gegebenenfalls durch Fluor, Chlor, Brom, $C_1$-$C_4$-Alkyl und/oder $C_1$-$C_4$-Alkoxy substituiertes Benzyl, für gegebenenfalls durch Fluor, Chlor, Brom, Cyano, Nitro, $C_1$-$C_4$-Alkyl und/oder $C_1$-$C_4$-Alkoxy substituiertes Phenyl, für Naphthyl, für jeweils gegebenenfalls durch Fluor, Chlor, Brom, Cyano, Nitro und/oder $C_1$-$C_4$-Alkyl substituiertes Pyridyl, Furyl oder Thienyl, für $C_1$-$C_6$-Alkoxy, Phenoxy, $C_1$-$C_6$-Alkylthio, Phenylthio, $C_1$-$C_6$-Alkyl-amino, Di-($C_1$-$C_4$)-alkylamino, $C_3C_6$-Cycloalkylamino oder für gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_4$-Alkoxy-carbonyl substituiertes Phenylamino steht und

$R^{10}$ für gegebenenfalls durch Fluor und/oder Chlor substituiertes $C_1$-$C_6$-Alkyl, für gegebenenfalls durch Fluor, Chlor, Brom, Cyano, Nitro, $C_1$-$C_4$-Alkyl, Trifluormethyl, $C_1$-$C_4$-Alkoxy, Difluormethoxy, Trifluormethoxy, $C_1$-$C_4$-Alkylthio, $C_1$-$C_4$- Alkylsulfinyl, $C_1$-$C_4$-Alkylsulfonyl, Di-($C_1$-$C_2$)alkylaminosulfonyl und/oder $C_1$-$C_4$-Alkoxy-carbonyl substituiertes Phenyl, für gegebenenfalls durch Chlor und/oder $C_1$-$C_4$-Alkyl substituiertes Naphtyl oder für jeweils gegebenenfalls durch Fluor, Chlor, Brom, Cyano, Nitro und/oder $C_1$-$C_4$-Alkyl substituiertes Pyridyl oder Thienyl steht,

$R^{11}$ für Wasserstoff, Chlor oder Brom steht und

$R^{12}$ für $C_1$-$C_6$-Alkyl steht.

Die Erfindung betrifft insbesondere Verbindungen der Formel (I), in welcher

$R^1$ für Wasserstoff, Fluor oder Chlor, insbesondere für Chlor steht,

$R^2$ für Wasserstoff, Fluor oder Chlor, insbesondere für Wasserstoff steht,

$R^3$ für Trifluormethyl steht,

$R^4$ für Wasserstoff, Fluor oder Chlor, insbesondere für Wasserstoff steht,

$R^5$ für Wasserstoff, Fluor oder Chlor, insbesondere für Fluor oder Chlor steht und

$R^6$ für die Gruppierungen

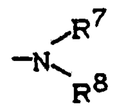

oder

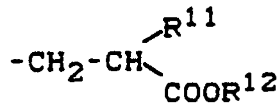

steht, worin

$R^7$ für Wasserstoff oder Acetyl steht und

$R^8$ für Wasserstoff, Methoxycarbonylethyl, Ethoxycarbonylethyl, die Gruppierung $-CO-R^9$ oder die Gruppierung $-SO_2-R^{10}$ steht, wobei

$R^9$ für gegebenenfalls durch Chlor, Brom, Methoxy oder Ethoxy substituiertes $C_1$-$C_4$-Alkyl, für Cyclopropyl, für Benzyl, für gegebenenfalls durch Fluor, Chlor, Brom, Methyl, Ethyl, Methoxy und/oder Ethoxy substituiertes Phenyl, für $C_1$-$C_4$-Alkoxy, Phenoxy, $C_1$-$C_4$-Alkylamino, Dimethylamino, Diethylamino, Cyclopentylamino, Cyclohexylamino oder für gegegebenenfalls durch Fluor, Chlor, Methyl, Ethyl, Trifluormethyl, Methoxy und/oder Ethoxy substituiertes Phenylamino steht und

$R^{10}$ für gegebenenfalls durch Fluor und/oder Chlor substituiertes $C_1$-$C_4$-Alkyl, für gegebenenfalls durch Fluor, Chlor, Brom, Methyl, Trifluormethoxy, Dimethylaminosulfonyl und/oder Methoxycarbonyl substituiertes Phenyl steht,

$R^{11}$ für Wasserstoff, Chlor oder Brom steht und

$R^{12}$ für $C_1$-$C_4$-Alkyl steht.

Beispiele für die erfindungsgemäßen Verbindungen der Formel (I) sind in der nachstehenden Tabelle 1 aufgeführt (vgl. auch die Herstellungsbeispiele).

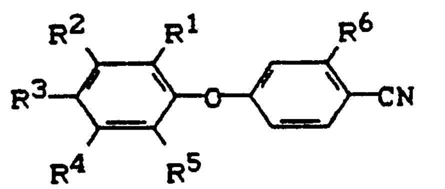

(I)

Tabelle 1: Beispiele für die Verbindungen der Formel (I)

| $R^1$ | $R^2$ | $R^3$ | $R^4$ | $R^5$ | $R^6$ |
|---|---|---|---|---|---|
| H | H | $CF_3$ | H | H | $NH_2$ |
| H | H | $CF_3$ | H | H | $-CH_2CH_2COOCH_3$ |
| Cl | H | $CF_3$ | H | H | $NH_2$ |
| Cl | H | $CF_3$ | H | H | $-CH_2CH_2COOCH_3$ |
| Cl | H | $CF_3$ | H | Cl | $NH_2$ |
| Cl | H | $CF_3$ | H | Cl | $-CH_2CH_2COOCH_3$ |
| Cl | H | $CF_3$ | Cl | Cl | $NH_2$ |
| Cl | H | $CF_3$ | Cl | Cl | $-CH_2CH_2COOCH_3$ |
| Cl | H | $CF_3$ | F | Cl | $NH_2$ |
| Cl | H | $CF_3$ | F | Cl | $-CH_2CH_2COOCH_3$ |

Tabelle 1 - Fortsetzung

| $R^1$ | $R^2$ | $R^3$ | $R^4$ | $R^5$ | $R^6$ |
|---|---|---|---|---|---|
| Cl | H | $CF_3$ | H | F | $NH_2$ |
| Cl | H | $CF_3$ | H | F | $-CH_2CH_2COOCH_3$ |
| F | H | $CF_3$ | H | F | $NH_2$ |
| Cl | H | $CF_3$ | H | Cl | $-NH-COCH_3$ |
| Cl | H | $CF_3$ | H | Cl | $-NH-COC_2H_5$ |
| Cl | H | $CF_3$ | H | Cl | $-NH-COC_3H_7$ |
| Cl | H | $CF_3$ | H | Cl | $-NH-COCH(CH_3)_2$ |
| Cl | H | $CF_3$ | H | Cl | $-NH-COC_4H_9$ |
| Cl | H | $CF_3$ | H | Cl | $-NH-COCH_2CH(CH_3)_2$ |
| Cl | H | $CF_3$ | H | Cl | $-NH-COC(CH_3)_3$ |
| Cl | H | $CF_3$ | H | Cl | $-NH-CH_2CH_2COOCH_3$ |
| Cl | H | $CF_3$ | H | Cl | $-NH-CH_2CH_2COOC_2H_5$ |
| Cl | H | $CF_3$ | H | Cl | $-N(COCH_3)_2$ |
| Cl | H | $CF_3$ | H | Cl | $-NH-SO_2CH_3$ |
| Cl | H | $CF_3$ | H | Cl | $-NH-SO_2C_2H_5$ |
| Cl | H | $CF_3$ | H | Cl | $-NH-SO_2CH_2Cl$ |
| Cl | H | $CF_3$ | H | Cl | $-NH-SO_2CF_3$ |
| Cl | H | $CF_3$ | H | Cl | $-NH-COOCH_3$ |
| Cl | H | $CF_3$ | H | Cl | $-NH-COOC_2H_5$ |
| Cl | H | $CF_3$ | H | Cl | $-NH-CONHCH_3$ |
| Cl | H | $CF_3$ | H | Cl | $-NH-CONHC_4H_9$ |
| Cl | H | $CF_3$ | H | Cl | $-NH-CO-N(CH_3)_2$ |
| Cl | H | $CF_3$ | H | Cl | $-NH-COCH_2Cl$ |

Tabelle 1 - Fortsetzung

| $R^1$ | $R^2$ | $R^3$ | $R^4$ | $R^5$ | $R^6$ |
|---|---|---|---|---|---|
| Cl | H | $CF_3$ | H | Cl | $-NH-COCHCl_2$ |
| Cl | H | $CF_3$ | H | Cl | $-NH-COCH_2OCH_3$ |
| Cl | H | $CF_3$ | H | Cl | —NH—CO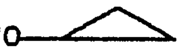 |
| Cl | H | $CF_3$ | H | Cl | $-NH-COCH_2-$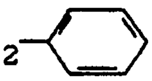 |
| Cl | H | $CF_3$ | H | Cl | —NH—CO—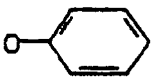 |
| Cl | H | $CF_3$ | H | Cl | —NH—CO—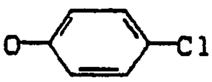 |
| Cl | H | $CF_3$ | H | Cl | —NH—CO—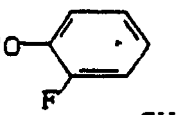 |
| Cl | H | $CF_3$ | H | Cl | —NH—CO—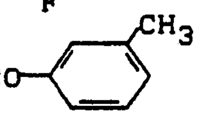 |
| Cl | H | $CF_3$ | H | Cl | —NH—COO—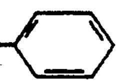 |
| Cl | H | $CF_3$ | H | Cl | —NH—CO—NH—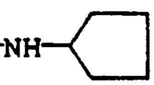 |
| Cl | H | $CF_3$ | H | Cl | —NH—CO—NH—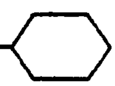 |
| Cl | H | $CF_3$ | H | Cl | —NH—CO—NH—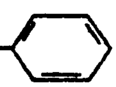 |
| Cl | H | $CF_3$ | H | Cl | —NH—CO—NH—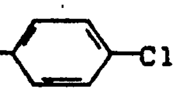 |
| Cl | H | $CF_3$, | H | Cl | —NH—CO—NH—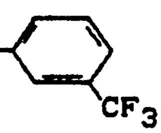 |

Tabelle 1 - Fortsetzung

| $R^1$ | $R^2$ | $R^3$ | $R^4$ | $R^5$ | $R^6$ |
|---|---|---|---|---|---|
| Cl | H | $CF_3$ | H | Cl | $-NH-SO_2-$ 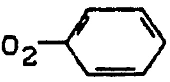 |
| Cl | H | $CF_3$ | H | Cl | $-NH-SO_2-$ 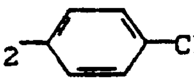 $-CH_3$ |
| Cl | H | $CF_3$ | H | Cl | $-CH_2CH_2COO_2H_5$ |
| Cl | H | $CF_3$ | H | Cl | $-CH_2\underset{Cl}{\underset{\vert}{C}}HCOOCH_3$ |
| Cl | H | $CF_3$ | H | Cl | $-CH_2\underset{Br}{\underset{\vert}{C}}HCOOCH_3$ |
| Cl | H | $CF_3$ | H | Cl | $-CH_2\underset{Cl}{\underset{\vert}{C}}HCOOC_2H_5$ |
| Cl | H | $CF_3$ | H | F | $-NH-COCH_3$ |
| Cl | H | $CF_3$ | H | F | $-NH-COC_4H_9$ |
| Cl | H | $CF_3$ | H | F | $-NH-CO-CH_2CH(CH_3)_2$ |
| Cl | H | $CF_3$ | H | F | $-NHCH_2CH_2COOCH_3$ |
| Cl | H | $CF_3$ | H | F | $-NHCH_2CH_2COOC_2H_5$ |
| Cl | H | $CF_3$ | H | F | $-NH-SO_2CH_3$ |
| Cl | H | $CF_3$ | H | F | $-NH-COCHCl_2$ |
| Cl | H | $CF_3$ | H | F | $-CH_2CH_2COOC_2H_5$ |
| Cl | H | $CF_3$ | H | F | $-CH_2\underset{Cl}{\underset{\vert}{C}}HCOOCH_3$ |
| Cl | H | $CF_3$ | H | F | $-CH_2\underset{Br}{\underset{\vert}{C}}HCOOCH_3$ |
| Cl | H | $CF_3$ | H | F | $-CH_2\underset{Cl}{\underset{\vert}{C}}HCOOC_2H_5$ |

Verwendet man für das erfindungsgemäße Verfahren (a) beispielsweise 3,4-Dichlor-benzotrifluorid und 2-Amino-4-hydroxy-benzonitril als Ausgangsstoffe, so kann der Reaktionsablauf durch das folgende Formelschema wiedergegeben werden:

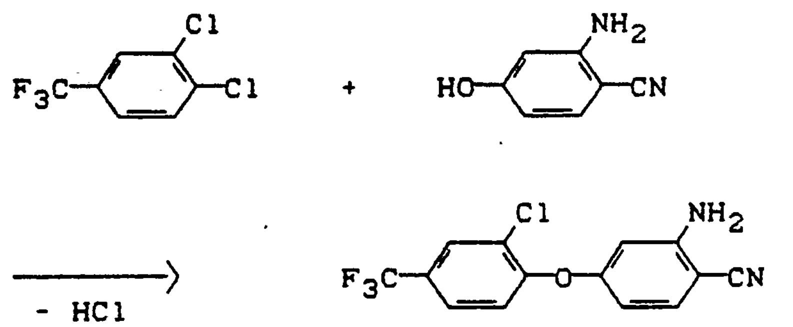


Verwendet man für das erfindungsgemäße Verfahren (b) beispielsweise 2-Amino-4-(2,6-difluor-4-trifluormethyl-phenoxy)-benzonitril und Dichloracetylchlorid als Ausgangsstoffe, so kann der Reaktionsablauf durch das folgende Formelschema wiedergegeben werden:

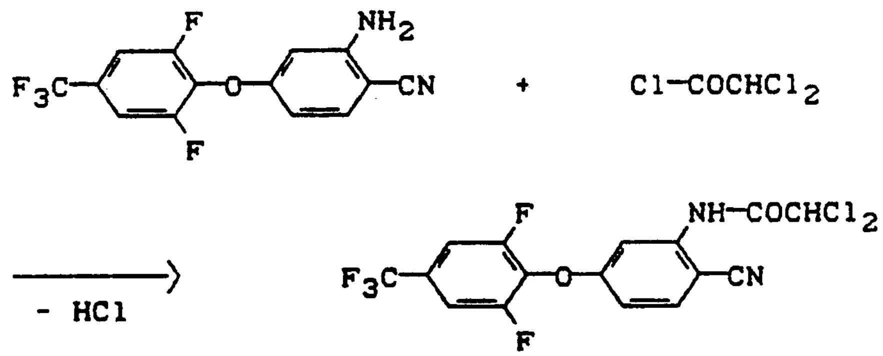


Verwendet man für das erfindungsgemäße Verfahren (c) beispielsweise 2-Amino-4-(2,3,6-Trichlor-4-trifluormethyl-phenoxy)-benzonitril und Acrylsäurebutylester als Ausgangsstoffe, so kann der Reaktionsablauf durch das folgende Formelschema wiedergegeben werden:

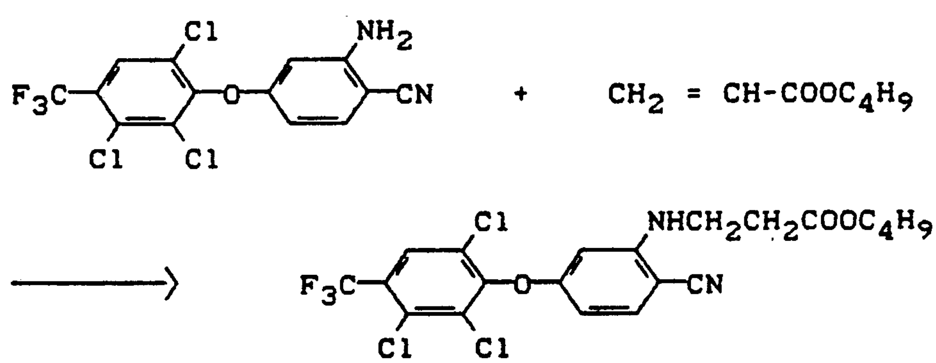

Verwendet man für das erfindungsgemäße Verfahren (d) beispielsweise 2-Amino-4-(2-Chlor-6-fluor-4-trifluormethyl-phenoxy)-benzonitril, Natriumnitrit und Salzsäure sowie anschließend Acrylsäure-isopropylester als Ausgangsstoffe, so kann der Reaktionsablauf durch das folgende Formelschema wiedergegeben werden:

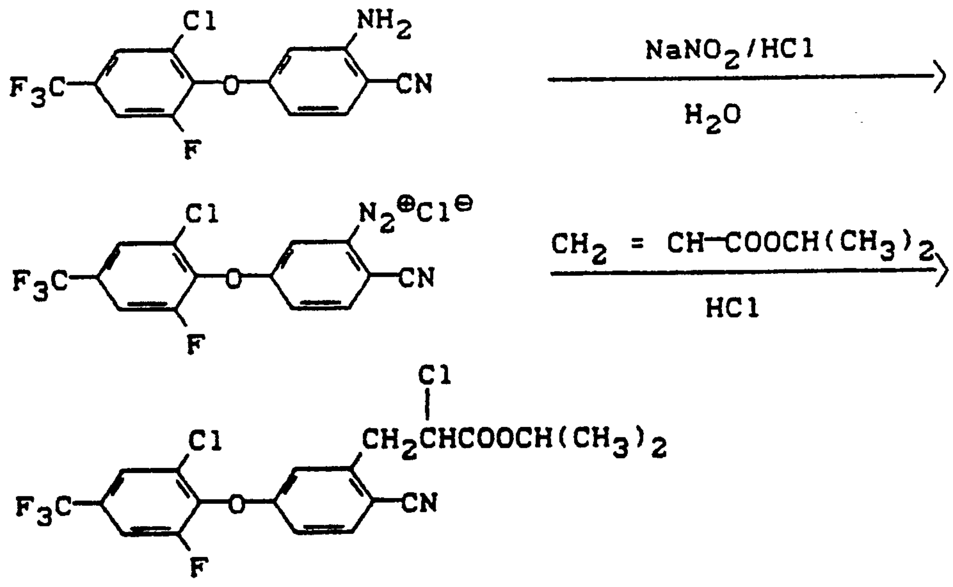


Verwendet man für das erfindungsgemäße Verfahren (e) beispielsweise 2-Chlor-3-[2-cyano-5-(2,6-dichlor-4-tri fluormethyl-phenoxy)-phenyl]-propionsäure-ethylester und Tributylzinnhydrid als Ausgangsstoffe, so kann der Reaktionsablauf durch das folgende Formelschema wiedergegeben werden:

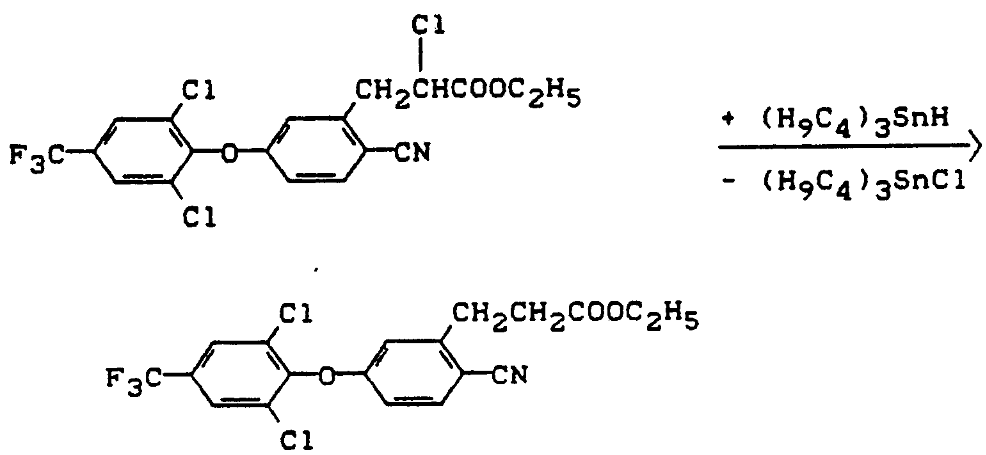


Die beim erfindungsgemäßen Verfahren (a) zur Herstellung von Verbindungen der Formel (I) als Ausgangsstoffe zu verwendenden Halogen-benzol-Derivate sind durch die Formel (II) allgemein definiert.

In Formel (II) haben $R^1$, $R^2$, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden und X steht vorzugsweise für Fluor oder Chlor.

Als Beispiele für die Ausgangsstoffe der Formel (II) seien genannt:
3,4-Dichlor-benzotrifluorid, 3,4,5-Trichlor-benzotrifluorid, 3,4-Dichlor-5-fluor-benzotrifluorid, 2,3,4,5-Tetrachlor-benzotrifluorid, 3,5-Dichlor-2,4-difluor-benzotrifluorid und 3-Chlor-4,5-difluor-benzotrifluorid.

Die Verbindungen der Formel (II) sind bekannt und/oder können nach an sich bekannten Verfahren hergestellt werden (vgl. J. Chem. Soc. 1969, 211-217; ibid. 1971, FR-A 2 538 380 (Chem. Abstracts 102 (1985), 61914x); EP-A 180 057; US-P 4 388 472).

Das bei Verfahren (a) weiter als Ausgangsstoff einzusetzende 2-Amino-4-hydroxy-benzonitril der Formel (III) ist bereits bekannt (vgl. Synthesis 1985, 778 - 779).

Die bei den erfindungsgemäßen Verfahren (b), (c) und (d) als Ausgangsstoffe zu verwendenden Verbindungen sind durch die Formel (I) mit der Maßgabe, daß $R^6$ für Amino steht, allgemein definiert. In diesem Fall haben die Reste $R^1$, $R^2$, $R^3$, $R^4$ und $R^5$ vorzugsweise bzw. insbesondere diejenigen Bedeutungen, die bereits im Rahmen der Beschreibung der erfindungsgemäßen Verbindungen der Formel (I)

vorzugsweise bzw. als insbesondere bevorzugt angegeben wurden.

Beispiele für diese Verbindungen sind Tabelle 1 ($R^6$:$NH_2$) zu entnehmen. Diese Ausgangsstoffe sind erfindungsgemäße, neue Verbindungen; sie können nach dem erfindungsgemäßen Verfahren (a) hergestellt werden.

Die beim erfindungsgemäßen Verfahren (b) weiter als Ausgangsstoffe zu verwendenden Verbindungen sind durch die Formeln (IV) und (V) allgemein definiert In diesen Formeln haben $R^9$ und $R^{10}$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^9$ und $R^{10}$ angegeben wurden und $X^1$ wie auch $X^2$ stehen vorzugsweise für Fluor, Chlor oder Brom, insbesondere für Chlor.

Als Beispiele für die Ausgangsstoffe der Formeln (IV) und (V) seien genannt:
Acetylchlorid, Propionylchlorid, Butyroylchlorid, Isobutyroylchlorid, Valeroylchlorid, Isovaleroylchlorid, Chloracetylchlorid, Dichloracetylchlorid, Trichloracetylchlorid, 2-Chlorpropionylchlorid, 2-Brompropionylchlorid, Methoxyacetylchlorid, Ethoxyacetylchlorid, 2-Methoxypropionylchlorid, 2-Ethoxypropionylchlorid, Cyclopropancarbonsäurechlorid, Phenylacetylchlorid, Benzoylchlorid, 2-Fluor-, 4-Fluor-, 2 Chlor-, 4-Chlor-, 2-Brom-, 4-Brom-, 2-Methyl-, 2-Ethyl-, 3-Methyl-, 4-Methyl-, 4-Ethyl-, 3-Methoxy-, 4-Methoxy- und 4-Ethoxybenzoylchlorid, Chlorameisensäure-methylester, -ethylester, -propylester und -butylester, Chlorameisensäurephenylester, Dimethylcarbamidsäurechlorid, Methan-, Ethan-, Propan-, Butan-, Chlormethan-, Trifluormethan-, 2-Chlor-ethan- und Perfluorbutan-sulfonsäurechlorid, Benzolsulfonsäurechlorid, 2-Chlor-, 3-Chlor-, 4-Chlor-, 2,4-Dichlor-, 2,5-Dichlor-, 2-Fluor-, 4-Fluor-, 2-Brom-, 4-Brom-, 2-Methyl-, 3-Methyl-, 4-Methyl-, 2-Trifluormethyl-, 2-Methoxy-, 4-Methoxy-, 2-Difluormethoxy-, 2- Trifluormethoxy-, 4-Trifluormethoxy-, 2-Dimethylaminosulfonyl-, 2-Methoxycarbonyl- und 4-Methoxycarbonylbenzolsulfonsäurechlorid.

Die Ausgangsstoffe der Formel (IV) und (V) sind bekannte Verbindungen.

Die bei den erfindungsgemäßen Verfahren (c) und (d) als Ausgangsstoffe zu verwendenden Acrylsäureester sind durch die Formel (VI) allgemein definiert. In Formel (VI) steht $R^{12}$ vorzugsweise für $C_1$-$C_6$-Alkyl, insbesondere für $C_1$-$C_4$-Alkyl.

Als Beispiele für die Ausgangsstoffe der Formel (VI) seien genannt:
Acrylsäure-methylester, -ethylester, -propylester und -butylester.

Die Verbindungen der Formel (VI) sind bekannte organische Synthesechemikalien.

Die beim erfindungsgemäßen Verfahren (e) als Ausgangsstoffe zu verwendenden Verbindungen sind durch die Formel (I) mit der Maßgabe, daß $R^6$ für die Gruppierung

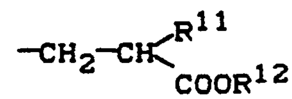

steht, allgemein definiert. In diesem Fall haben die Reste $R^1$, $R^2$, $R^3$, $R^4$, $R^5$ und $R^{12}$ vorzugsweise bzw. insbesondere diejenigen Bedeutungen, die bereits im Rahmen der Beschreibung der erfindungsgemäßen Verbindungen der Formel (I) vorzugsweise bzw. als insbesondere bevorzugt angegeben wurden und $R^{11}$ steht vor zugsweise für Chlor oder Brom.

Beispiele für diese Verbindungen sind Tabelle 1 zu entnehmen. Diese Ausgangsstoffe sind erfindungsgemäße, neue Verbindungen; sie können nach dem erfindungsgemäßen Verfahren (d) hergestellt werden.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z B. Acetonitril und Propionitril, Amide wie z B. Dimethylformamid, Dimethylacetamid und N-Methyl-pyrrolidon sowie Dimethylsulfoxid, Tetramethylensulfon und Hexamethylphosphorsäuretriamid.

Hiervon werden die aprotisch polaren Lösungsmittel, wie z. B. Aceton, Acetonitril, Methylethylketon, Propionitril, Methylisobutylketon, Methylisopropylketon, Dimethylformamid, Dimethylacetamid, N-Methylpyrrolidon und Dimethylsulfoxid, besonders bevorzugt.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 200 °C, vorzugsweise bei Temperaturen zwischen 20 °C und 120 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säure akzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Das erfindungsgemäße Verfahren (b) zur Herstellung der neuen Verbindungen der Formel (I) wird gegebenenfalls unter Verwendung von Verdünnungsmitteln durchgeführt. Als Verdünnungsmittel kommen dabei alle inerten organischen Lösungsmittel in Frage, wie sie oben für das erfindungsgemäße Verfahren (a) angegeben sind.

Verfahren (b) wird gegebenenfalls in Gegenwart eines Säureakzeptors durchgeführt. Als Säureakzeptoren kommen hierbei praktisch alle üblicherweise für derartige Umsetzungen verwendbaren Säurebindemittel in Frage, wie sie oben für das erfindungsgemäße Verfahren (a) angegeben sind.

Hiervon werden die oben genannten aliphatischen, aromatischen oder heterocyclischen Amine, wie z. B. Pyridin und DABCO, besonders bevorzugt.

Die Reaktionstemperaturen können bei dem erfindungsgemäßen Verfahren (b) in einem größeren Bereich variiert werden. Im allgemeinen arbeitet man bei Temperaturen zwischen -20 °C und +150 °C, vorzugsweise bei Temperaturen zwischen 0 °C und 100 °C.

Das erfindungsgemäße Verfahren (b) wird im allgemeinen unter Normaldruck durchgeführt. Es ist jedoch auch mög lich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) nach üblichen Methoden.

Das erfindungsgemäße Verfahren (c) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage, wie sie oben für das erfindungsgemäße Verfahren (a) angegeben sind. Hiervon werden die oben angegebenen aprotisch polaren Lösungsmittel besonders bevorzugt.

Verfahren (c) wird gegebenenfalls in Gegenwart eines basischen Katalysators durchgeführt. Geeignete basische Katalysatoren sind hierbei Alkalimetall-hydroxide oder -alkoholate, wie Natrium- oder Kaliumhydroxid, -methylat, -ethylat oder -tert-butylat, Alkalimetallsalze von Carbonsäuren, vie z. B. Natrium- oder Kalium -acetat, sowie basische Stickstoffverbindungen, wie Diethylamin oder Piperidin.

Die Reaktionstemperaturen können bei dem erfindungsgemäßen Verfahren (c) in einem größeren Bereich variiert werden. Im allgemeinen arbeitet man bei Temperaturen zwischen 0 °C und 150 °C, vorzugsweise bei Temperaturen zwischen 20 °C und 120 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines basischen Katalysators durchgeführt, und das Reaktionsgemisch wird mehrere Stunden bei der jeweils erforderlichen Temperatur gerührt. Die Aufarbeitung erfolgt bei dem erfindungsgemäßen Verfahren (c) nach üblichen Methoden.

Das erfindungsgemäße Verfahren (d) zur Herstellung von Verbindungen der Formel (I) wird unter Einsatz eines Hydrogenhalogenids ($HX^3$) durchgeführt. Als Beispiele hierfür seien Hydrogenfluorid, Hydrogenchlorid, Hydrogenbromid und Hydrogeniodid genannt. Hydrogenchlorid und Hydrogenbromid werden vorzugsweise eingesetzt.

Verfahren (d) wird vorzugsweise unter Verwendung eines organischen Lösungsmittels durchgeführt. Insbesondere geeignet sind Ether, wie z B. Glycoldimethylether und Diglycoldimethylether, Tetrahydrofuran und Dioxan, Ketone, wie z. B. Aceton und Methylethylketon, sowie Amide wie z. B. Dimethylformamid.

Verfahren (d) wird weiter vorzugsweise in Gegenwart von Katalysatoren duchgeführt. Als solche kommen insbesondere Kupfer und Kupferverbindungen, wie z. B. Kupfer(I)-chlorid, Kupfer(II)-chlorid, Kupfer(I)-bromid, Kupfer(I)-iodid, Kupfer(II)-sulfat und Kupfer(II)-nitrat in Betracht.

Die Reaktionstemperaturen können bei dem erfindungsgemäßen Verfahren (d) in einem größeren Bereich variiert werden. Im allgemeinen arbeitet man bei Temperaturen zwischen -20 °C und +80 °C, vorzugsweise bei Temperaturen zwischen 0 °C und 60 °C.

Verfahren (d) wird im allgemeinen bei Normaldruck durchgeführt.

Zur Durchführung des erfindungsgemäßen Verfahrens (d) setzt man je Mol Phenoxyphenylaminoverbindung der Formel (I) im allgemeinen zwischen 0,8 und 2,5 Mol, vorzugsweise zwischen 1,1 und 2,0 Mol, Natriumnitrit oder Kaliumnitrit, zwischen 2 und 50 Mol, vorzugsweise zwischen 5 und 25 Mol, Hydrogenhalogenid, und zwischen 1 und 3 Mol, vorzugsweise zwischen 1,5 und 2,5 Mol, Acrylsäure-Derivat der Formel (VI) ein.

Verfahren (d) kann unter den üblichen Bedingungen der "Meerwein-Arylierung" durchgeführt werden. In einer bevorzugten Ausführungsform von Verfahren (d) wird zunächst die Ausgangsverbindung der Formel (I) in einem Verdünnungsmittel, welches zumindest Wasser und ein Hydrogenhalogenid enthält, verrührt und unter Kühlen mit einer wäßrigen Lösung von Natriumnitrit oder Kaliumnitrit diazotiert. Dann wird das Acrylsäure-Derivat der Formel (VI) und gegebenenfalls der Katalysator zum Reaktionsgemisch gegeben. Wenn - gegebenenfalls nach leichtem Erwärmen - die Stickstoff-Entwicklung abgeklungen ist, kann nach üblichen Methoden aufgearbeitet werden.

Das erfindungsgemäße Verfahren (e) zur Herstellung von Verbindungen der Formel (I) wird unter Einsatz eines Dehalogenierungsmittels durchgeführt. Es können hierbei die üblichen zur Dehalogenierung geeigneten Substanzen, wie z. B. Tributylzinnhydrid, verwendet werden.

Verfahren (e) wird gegebenenfalls in Gegenwart eines Katalysators durchgeführt. Als Katalysatoren kommen hierbei als Radikalstarter gebräuchliche Substanzen, wie z. B. Azo-bis-isobutyronitril, in Betracht.

Verfahren (e) wird vorzugsweise in Gegenwart eines Verdünnungsmittels durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, wie Pentan, Hexan, Heptan, Cyclohexan, Methylcyclohexan, Benzol, Toluol, Xylol, Chlorbenzol und o-Dichlorbenzol, sowie Ether, wie Diethylether, Dipro pylether, Diisopropylether, Dibutylether, Diisobutylether, Glycoldimethylether, Diglycoldimethylether, Tetrahydrofuran und Dioxan.

Die Reaktionstemperaturen können bei dem erfindungsgemäßen Verfahren (e) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Katalysators durchgeführt, und das Reaktionsgemisch wird mehrere Stunden bei der jeweils erforderlichen Temperatur gerührt. Die Aufarbeitung erfolgt bei dem erfindungsgemäßen Verfahren (e) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verste hen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca,

Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen eignen sich insbesondere zur selektiven Bekämpfung von dikotylen Unkräutern in monokotylen Kulturen, vor allem im Nachlauf-Verfahren.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kom men im wesentlichen in Frage: Aromaten, wie Xylol, Toluol oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy methylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl )-1 ,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Di chlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on2,2-dioxid (BENTAZON); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); N-Phosphonomethyl-glycin (GLYPHOSATE); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele:

### Beispiel 1

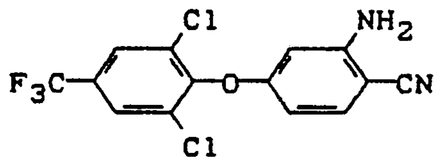

[Verfahren (a)]

13,4 g (0,1 Mol) 2-Amino-4-hydroxy-benzonitril werden in 200 ml Dimethylsulfoxid vorgelegt und nach Zugabe von 4,0 g (0,1 Mol) Natriumhydroxid wird das Gemisch 30 Minuten bei 50 °C gerührt. Anschließend wird eine Lösung von 24,9 g (0,1 Mol) 3,4,5-Trichlor-benzotrifluorid in 100 ml Dimethylsulfoxid bei 50 °C unter Rühren tropfenweise dazugegeben. Dann wird das Reaktionsgemisch noch 5 Stunden bei 50 °C und weitere 2 Stunden bei 90 °C gerührt, nach Abkühlen in 1 l Wasser gegossen und das Produkt mit Chloroform extrahiert. Die organische Phase wird mit Magnesiumsulfat getrocknet und filtriert. Vom Filtrat wird das Lösungsmittel im Wasserstrahlvakuum sorgfältig abdestilliert.

Man erhält 29,5 g (85 % der Theorie) 2-Amino-4-(2,6-dichor-4-trifluormethyl-phenoxy)-benzonitril als amorphen Rückstand.

$^1$H-NMR($CDCl_3$, δ): 7,63 [s, 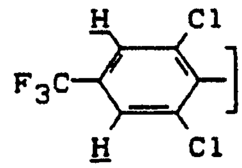 ];

7,25 [d, 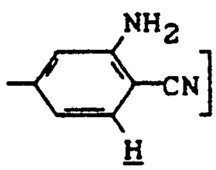 ]; 6,11 [dd, 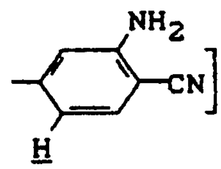 ];

6,08 [d, 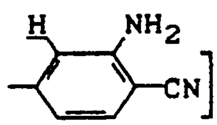 ].

Beispiel 2

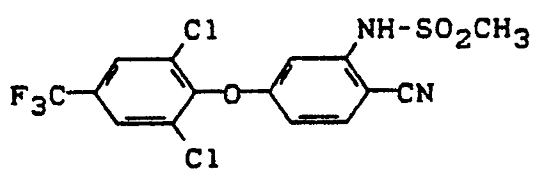

[Verfahren (b)]

Eine Mischung aus 3,47g (0,01 Mol) 2-Amino-4-(2,6-dichlor-4-trifluormethyl-phenoxy)-benzonitril, 10 ml Methansulfonsäurechlorid und 1 g Pyridin wird 15 Stunden bei 60 °C gerührt. Dann wird die Reaktionsmischung in 50 ml Wasser gegossen und das Produkt mit Chloroform extrahiert. Die organische Phase wird mit Magnesiumsulfat getrocknet und filtriert. Vom Filtrat wird das Lösungsmittel im Wasserstrahlvakuum

abdestilliert und der Rückstand durch Säulenchromatographie (Kieselgel; Hexan/Essigsäureethylester 4 : 1) gereinigt. Man erhält 2,2 g (52 % der Theorie) 2-Methylsulfonylamino-4-(2,6- dichlor-4-trifluormethyl-phenoxy)-benzonitril als amorphes Produkt.

$^1$H-NMR($D_6$-DMSO, δ): 8,22 [s, 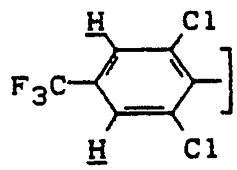 ];

7,83 [d, 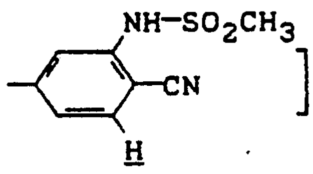 ]; 7,09 [d, 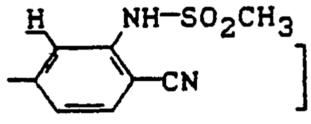 ]

6,90 [dd, 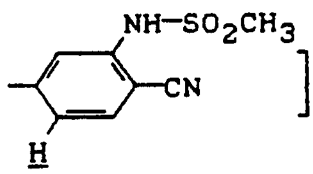 ]; 3,12(s, $SO_2CH_3$).

Beispiel 3

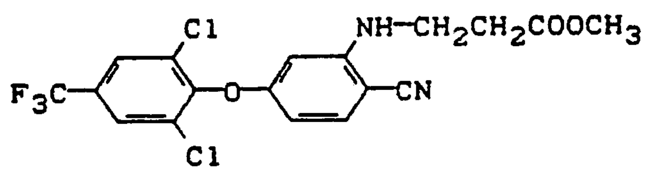

[Verfahren (c)]

Eine Mischung aus 4,16 g (0,012 Mol) 2-Amino-4-(2,6-dichlor-4-trifluormethyl-phenoxy)-benzonitril, 0,3 g Natriumacetat, 1,03 g (0,012 Mol) Acrylsäuremethylester und 100 ml Dimethylsulfoxid wird 8 Stunden bei 100 °C gerührt und dann in 300 ml Wasser gegossen. Das Produkt wird mit Chloroform extrahiert, die organische Phase mit Magnesiumsulfat getrocknet und filtriert Vom Filtrat wird das Lösungsmittel im Wasserstrahlvakuum abdestilliert und der Rückstand durch Säulenchromatographie (Kieselgel; Ether/Petrolether 1 : 2) gereinigt. Man erhält 0,62 g (12 % der Theorie) 2-(2-Methoxycarbonylethylamino)-4-(2,6-dichlor-4-trifluormethyl-phenoxy)-benzonitril als amorphes Produkt.

$^1H$-NMR($CDCl_3$, δ): 7,70 [s, 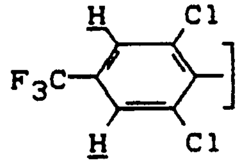];

3,72(s, $COOC\underline{H}_3$); 3,50(q, $-NH-C\underline{H}_2CH_2COOCH_3$);

2,68(t, $-NH-CH_2-C\underline{H}_2COOCH_3$).

Beispiel 4

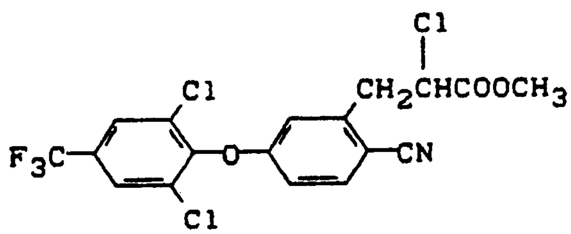

[Verfahren (d)]

Zu 6,9 g (0,02 Mol) 2-Amino-4-(2,6-dichlor-4-trifluormethyl-phenoxy)-benzonitril in 200 ml Aceton gibt man 50 ml 18%ige Salzsäure und anschließend tropfenweise unter Rühren und Kühlen auf 0 °C bis 5 °C eine Lösung von 2,0 g (0,03 Mol) Natriumnitrit in 10 ml Wasser. Das Reaktionsgemisch wird 30 Minuten bei 0 °C bis 5 °C gerührt und anschließend werden 8,6 g (0,1 Mol) Acryl säuremethylester und eine Lösung von 2 g (0,02 Mol) Kupfer(I)-chlorid in 5 ml konzentrierter Salzsäure dazugegeben. Nach 2 Stunden Rühren bei 5 °C wird mit 200 ml Wasser verdünnt, das Produkt mit Chloroform extrahiert, die organische Phase mit Magnesiumsulfat getrocknet und filtriert. Vom Filtrat wird das Lösungsmittel im Wasserstrahlvakuum abdestilliert und der Rückstand durch Säulenchromatographie (Kieselgel, Hexan/Essigsäureethylester 4 : 1) gereinigt.

Man erhält 3,8 g (42 % der Theorie) 2-(2-Chlor-2-methoxycarbonyl-ethyl)-4-(2,6-dichlor-4-trifluormethylphenoxy)-benzonitril als amorphes Produkt.

$^1H$-NMR($CDCl_3$, δ): 7,72 [s, 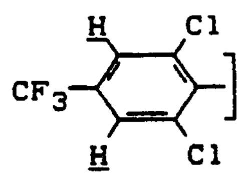];

4,58(X von ABX-Signal, 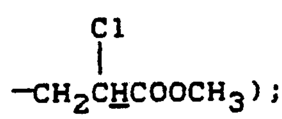);

3,80(s, $COOC\underline{H}_3$); 3,45(AB von ABX-Signal, 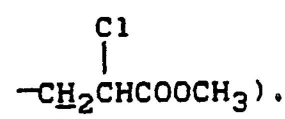).

Beispiel 5

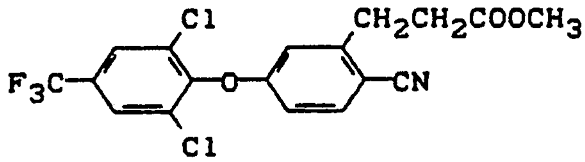

[Verfahren (e)]

3,0 g (0,0069 Mol) 2-(2-Chlor-2-methoxycarbonylethyl)-4-(2,6-dichlor-4-trifluormethyl-phenoxy)-benzonitril werden in 50 ml Toluol gelöst und 2,0 g (0,0069 Mol) Tributylzinnhydrid werden dazu gegeben. Nach Zugabe von 0,5 g Azo-bis-isobutyronitril wird das Reaktionsgemisch 10 Stunden bei 60 °C gerührt. Dann wird das Lösungsmittel im Wasserstrahlvakuum abdestilliert und der Rückstand durch Säulenchromatographie (Kieselgel, Methylenchlorid) gereinigt.

Man erhält 1,3 g (43 % der Theorie) 2-(2-Methoxycarbonyl-ethyl)-4-(2,6-dichlor-4-trifluormethyl-phenoxy)-benzonitril als amorphes Produkt.

$^1H$-NMR($CDCl_3$, δ): 7,61 [m, 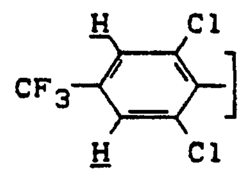 ];

7,43 [dd, 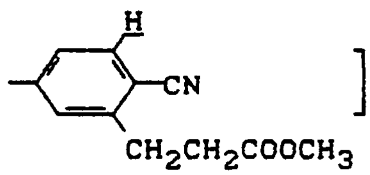 ];

6,87 [d, 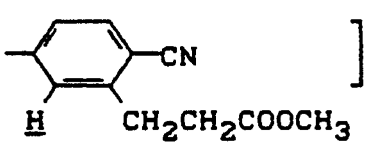 ];

6,79 [dd, 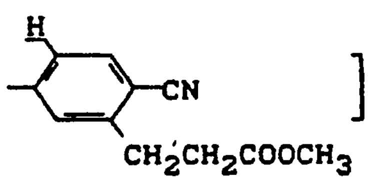 ];

3,67(s, $COOC\underline{H}_3$); 3,15(t, $-CH_2C\underline{H}_2COOCH_3$);
2,74(t, $-C\underline{H}_2CH_2COOCH_3$).

Analog zu den Beispielen 1 bis 5 und entsprechend der allgemeinen Beschreibung der erfindungsgemäßen Verfahren können die in der nachstehenden Tabelle 2 aufgeführten Verbindungen der Formel (I) hergestellt werden.

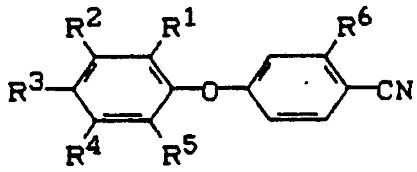

(I)

<u>Tabelle 2:</u> Herstellungsbeispiele für die Verbindungen der Formel (I)

| Beisp.-Nr. | $R^1$ | $R^2$ | $R^3$ | $R^4$ | $R^5$ | $R^6$ | Schmelzpunkt (°C) |
|---|---|---|---|---|---|---|---|
| 6 | Cl | H | $CF_3$ | H | H | $NH_2$ | |
| 7 | Cl | H | $CF_3$ | H | F | $NH_2$ | 63 |
| 8 | Cl | H | $CF_3$ | H | Cl | —NH—CO—NH— 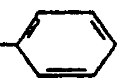 | 105 |
| 9 | Cl | H | $CF_3$ | H | Cl | $-N(COCH_3)_2$ | |
| 10 | Cl | H | $CF_3$ | H | Cl | —NH—CO— 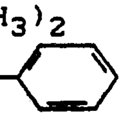 | 145 |
| 11 | Cl | H | $CF_3$ | H | Cl | $-NHCOCH_3$ | |
| 12 | Cl | H | $CF_3$ | H | Cl | —NH—CO—NH— 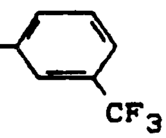 | 255 |
| 13 | Cl | H | $CF_3$ | H | Cl | —NH—CO—NH— 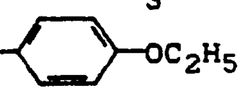 | 237 |

Tabelle 2 - Fortsetzung

| Beisp.-Nr. | $R^1$ | $R^2$ | $R^3$ | $R^4$ | $R^5$ | $R^6$ | Schmelzpunkt (°C) |
|---|---|---|---|---|---|---|---|
| 14 | Cl | H | $CF_3$ | H | Cl | $-NH-COO-$ 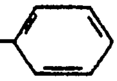 | |
| 15 | Cl | H | $CF_3$ | H | Cl | $-NH-CO-N(CH_3)_2$ | 103 |
| 16 | Cl | H | $CF_3$ | H | Cl | $-NH-CO-CH(CH_3)_2$ | |
| 17 | Cl | H | $CF_3$ | H | Cl | $-NH-CO-C_2H_5$ | 159 |
| 18 | Cl | H | $CF_3$ | H | Cl | $-NH-CO-C(CH_3)_3$ | |
| 19 | Cl | H | $CF_3$ | H | Cl | $-NH-CO-CH_2CH(CH_3)_2$ | 175 |
| 20 | Cl | H | $CF_3$ | H | Cl | $-CH_2\underset{\substack{\vert \\ Cl}}{C}HCOOCH_3$ | |

Anwendungsbeispiele

In den folgenden Anwendungsbeispielen wird die nachstehend aufgeführte Verbindung als Vergleichssubstanz herangezogen:

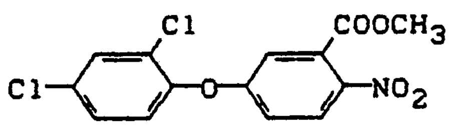

(A)

3-(2,4-Dichlor-phenoxy)-6-nitrobenzoesäure-methylester

(bekannt aus US-PS 36 52 645 und US-PS 3 776 715).

Beispiel A

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzübereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im

Vergleich zur Entwicklung der unbehandelten Kontrolle.
Es bedeuten:
0 % = keine Wirkung (wie unbehandelte Kontrolle)
100 % = totale Vernichtung

In diesem Test zeigen beispielsweise die Verbindungen gemäß den Herstellungsbeispielen (2) und (4) erheblich stärkere Wirkung als die bekannte Verbindung (A) gegen Unkräuter, wie z. B. Ipomoea, Sida, Sinapis und Viola.

Beispiel B

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert.

In diesem Test zeigen beispielsweise die Wirkstoffe gemäß den Herstellungsbeispielen (3) und (4) ein sehr starkes Austrocknen der Blätter und sehr starken Blattfall.

**Ansprüche**

1. Substituierte Phenoxybenzonitril-Derivate der Formel (I),

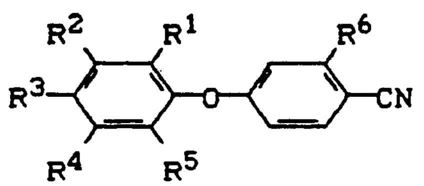

(I)

in welcher
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy oder Trifluormethylsulfonyl steht,
$R^4$ für Wasserstoff oder Halogen steht,
$R^5$ für Wasserstoff oder Halogen steht und
$R^6$ für die Gruppierungen

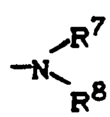

oder

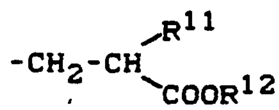

steht, worin
$R^7$ und $R^8$ gleich oder verschieden sind und unabhängig voneinander jeweils für Wasserstoff, Alkoxycarbonylethyl, die Gruppierung -CO-$R^9$ oder die Gruppierung -$SO_2$-$R^{10}$ stehen, wobei
$R^9$ für jeweils gegebenenfalls substituiertes Alkyl, Alkenyl, Alkinyl, Cycloalkyl, Cycloalkylalkyl, Benzyl, Phenyl, Naphthyl, Pyridyl, Furyl, Thienyl, Alkoxy, Phenoxy, Alkylthio, Phenylthio, Alkylamino, Dialkylamino, Cycloalkylamino oder Phenylamino steht und
$R^{10}$ für jeweils gegebenenfalls substituiertes Alkyl, Phenyl, Naphthyl, Pyridyl oder Thienyl steht,
$R^{11}$ für Wasserstoff oder Halogen steht und
$R^{12}$ für Alkyl steht.

2. Substituierte Phenoxybenzonitril-Derivate der Formel (I) gemäß Anspruch 1, in welcher
$R^1$ für Wasserstoff, Fluor, Chlor, Brom, Cyano oder Trifluormethyl steht,
$R^2$ für Wasserstoff, Fluor oder Chlor steht,
$R^3$ für Fluor, Chlor, Brom, Trifluormethyl, Trifluormethoxy oder Trifluormethylsulfonyl steht,
$R^4$ für Wasserstoff, Fluor oder Chlor steht,
$R^5$ für Wasserstoff, Fluor oder Chlor steht und
$R^6$ für die Gruppierungen

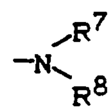

oder

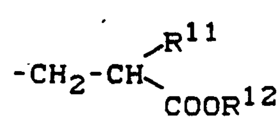

steht, worin
$R^7$ und $R^8$ gleich oder verschieden sind und unabhängig voneinander jeweils für Wasserstoff, $C_1$-$C_6$-Alkoxy-carbonyl-ethyl, die Gruppierung -CO-$R^9$ oder die Gruppierung -$SO_2$-$R^{10}$ stehen, wobei
$R^9$ für gegebenenfalls durch Fluor, Chlor, Brom, $C_1$-$C_4$-Alkoxy und/oder $C_1$-$C_4$-Alkoxy-carbonyl substituiertes $C_1$-$C_6$-Alkyl, für gegebenenfalls durch Fluor, Chlor und/oder Brom substituiertes $C_2$-$C_4$-Alkenyl, für $C_2$-$C_4$-Alkinyl, für gegebenenfalls durch Fluor, Chlor, Brom, $C_1$-$C_4$-Alkyl und/oder $C_1$-$C_4$-Alkoxy-carbonyl substituiertes $C_3$-$C_6$-Cycloalkyl, für gegebenenfalls durch Fluor, Chlor, Brom und/oder $C_1$-$C_4$-Alkyl substituiertes $C_3$-$C_6$-Cycloalkyl-$C_1$-$C_2$-alkyl, für gegebenenfalls durch Fluor, Chlor, Brom, $C_1$-$C_4$-Alkyl und/oder $C_1$-$C_4$-Alkoxy substituiertes Benzyl, für gegebenenfalls durch Fluor, Chlor, Brom, Cyano, Nitro, $C_1$-$C_4$-Alkyl und/oder $C_1$-$C_4$-Alkoxy substituiertes Phenyl, für Naphthyl, für jeweils gegebenenfalls durch Fluor, Chlor, Brom, Cyano, Nitro und/oder $C_1$-$C_4$-Alkyl substituiertes Pyridyl, Furyl oder Thienyl, für $C_1$-$C_6$-Alkoxy, Phenoxy, $C_1$-$C_6$-Alkylthio, Phenylthio, $C_1$-$C_6$-Alkylamino, Di-($C_1$-$C_4$)-alkylamino, $C_3$-$C_6$-Cycloalkylamino oder für gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_4$-Alkoxy-carbonyl substituiertes Phenylamino steht und
$R^{10}$ für gegebenenfalls durch Fluor und/oder Chlor substituiertes $C_1$-$C_6$-Alkyl, für gegebenenfalls durch Fluor, Chlor, Brom, Cyano, Nitro, $C_1$-$C_4$-Alkyl, Trifluormethyl, $C_1$-$C_4$-Alkoxy, Difluormethoxy, Trifluormethoxy, $C_1$-$C_4$-Alkylthio, $C_1$-$C_4$-Alkylsulfinyl, $C_1$-$C_4$-Alkylsulfonyl, Di-($C_1$-$C_2$)alkylaminosulfonyl und/oder $C_1$-$C_4$-Alkoxycarbonyl substituiertes Phenyl, für gegebenenfalls durch Chlor und/oder $C_1$-$C_4$-Alkyl substituiertes Naphtyl oder für jeweils gegebenenfalls durch Fluor, Chlor, Brom, Cyano, Nitro und/oder $C_1$-$C_4$-Alkyl substituiertes Pyridyl oder Thienyl steht,
$R^{11}$ für Wasserstoff, Chlor oder Brom steht und
$R^{12}$ für $C_1$-$C_6$-Alkyl steht.

3. Substituierte Phenoxybenzonitril-Derivate der Formel (I) gemäß Anspruch 1, in welcher
$R^1$ für Wasserstoff, Fluor oder Chlor steht,
$R^2$ für Wasserstoff, Fluor oder Chlor steht,
$R^3$ für Trifluormethyl steht,
$R^4$ für Wasserstoff, Fluor oder Chlor steht,
$R^5$ für Wasserstoff, Fluor oder Chlor steht,
$R^6$ für die Gruppierungen

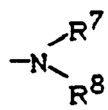

oder

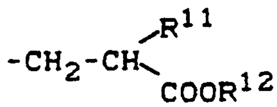

steht, worin
$R^7$ für Wasserstoff oder Acetyl steht und
$R^8$ für Wasserstoff, Methoxycarbonylethyl, Ethoxycarbonylethyl, die Gruppierung $-CO-R^9$ oder die Gruppierung $-SO_2-R^{10}$ steht, wobei
$R^9$ für gegebenenfalls durch Chlor, Brom, Methoxy oder Ethoxy substituiertes $C_1$-$C_4$-Alkyl, für Cyclopropyl, für Benzyl, für gegebenenfalls durch Fluor, Chlor, Brom, Methyl, Ethyl, Methoxy und/oder Ethoxy substituiertes Phenyl, für $C_1$-$C_4$-Alkoxy, Phenoxy, $C_1$-$C_4$-Alkylamino, Dimethylamino, Diethylamino, Cyclopentylamino, Cyclohexylamino oder für gegegebenenfalls durch Fluor, Chlor, Methyl, Ethyl, Trifluormethyl, Methoxy und/oder Ethoxy substituiertes Phenylamino steht und
$R^{10}$ für gegebenenfalls durch Fluor und/oder Chlor substituiertes $C_1$-$C_4$-Alkyl, für gegebenenfalls durch Fluor, Chlor, Brom, Methyl, Trifluormethoxy, Dimethylaminosulfonyl und/oder Methoxycarbonyl substituiertes Phenyl steht,
$R^{11}$ für Wasserstoff, Chlor oder Brom steht und
$R^{12}$ für $C_1$-$C_4$-Alkyl steht.

4. Verfahren zur Herstellung von substituierten Phenoxybenzonitril-Derivaten der Formel (I),

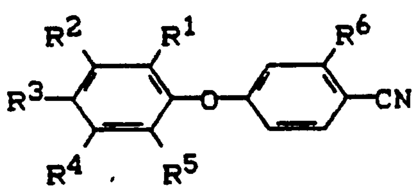

(I)

in welcher
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy oder Trifluormethylsulfonyl steht,
$R^4$ für Wasserstoff oder Halogen steht,
$R^5$ für Wasserstoff oder Halogen steht und
$R^6$ für die Gruppierungen

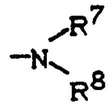

,

oder

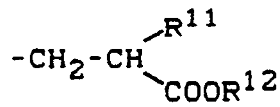

steht, worin

$R^7$ und $R^8$ gleich oder verschieden sind und unabhängig voneinander jeweils für Wasserstoff, Alkoxycarbonylethyl, die Gruppierung -CO-$R^9$ oder die Gruppierung -$SO_2$-$R^{10}$ stehen, wobei

$R^9$ für jeweils gegebenenfalls substituiertes Alkyl, Alkenyl, Alkinyl, Cycloalkyl, Cycloalkylalkyl, Benzyl, Phenyl, Naphthyl, Pyridyl, Furyl, Thienyl, Alkoxy, Phenoxy, Alkylthio, Phenylthio, Alkylamino, Dialkylamino, Cycloalkylamino oder Phenylamino steht und

$R^{10}$ für jeweils gegebenenfalls substituiertes Alkyl, Phenyl, Naphthyl, Pyridyl oder Thienyl steht,

$R^{11}$ für Wasserstoff oder Halogen steht und

$R^{12}$ für Alkyl steht,

dadurch gekennzeichnet, daß man

(a) für den Fall, daß in Formel (I) $R^6$ für Amino steht und $R^1$, $R^2$, $R^3$, $R^4$ und $R^5$ die oben angegebenen Bedeutungen haben,

Halogen-benzol-Derivate der allgemeinen Formel (II)

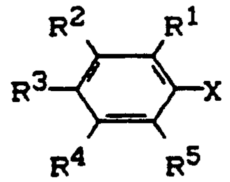

(II)

in welcher

$R^1$, $R^2$, $R^3$, $R^4$ und $R^5$ die oben angegebenen Bedeutungen haben und

X für Halogen steht,

mit 2-Amino-4-hydroxy-benzonitril der Formel (III)

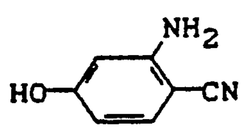

(III)

gegebenenfalls in Gegenwart eines Säureakzeptors und gegebenenfalls in Gegenwart eines Verdünnungsmittels umsetzt, oder daß man

(b) für den Fall, daß in Formel (I) $R^6$ für die Gruppierung

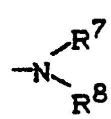

steht, worin $R^7$ und/oder $R^8$ für die Gruppierung -CO-$R^9$ oder die Gruppierung -$SO_2$-$R^{10}$ stehen, und $R^1$, $R^2$, $R^3$, $R^4$, $R^5$, $R^9$ und $R^{10}$ die oben angegebene Bedeutung haben,

Verbindungen der allgemeinen Formel (I), in welcher $R^6$ für Amino steht und $R^1$, $R^2$, $R^3$, $R^4$ und $R^5$ die oben angegebenen Bedeutungen haben,

mit Verbindungen der allgemeinen Formel (IV)

$X^1$-CO-$R^9$ (IV)

in welcher

$R^9$ die oben angegebene Bedeutung hat und
$X^1$ für Halogen steht,
oder mit Verbindungen der allgemeinen Formel (V)
$X^2-SO_2-R^{10}$ (V)
in welcher
$R^{10}$ die oben angegebene Bedeutung hat und
$X^2$ für Halogen steht,
gegebenenfalls in Gegenwart eines Säureakzeptors und gegebenenfalls in Gegenwart eines Verdünnungsmittels umsetzt, oder daß man

(c) für den Fall, daß in Formel (I) $R^6$ für die Guppierung

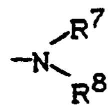

steht, worin $R^7$ und/oder $R^8$ für Alkoxycarbonylethyl stehen, und $R^1$, $R^2$, $R^3$, $R^4$ und $R^5$ die oben angegebenen Bedeutungen haben,
Verbindungen der allgemeinen Formel (I), in welcher $R^6$ für Amino steht und $R^1$, $R^2$, $R^3$, $R^4$ und $R^5$ die oben angegebenen Bedeutungen haben,
mit Acrylsäureestern der Formel (VI)
$CH_2=CH-COOR^{12}$ (VI)
in welcher
$R^{12}$ für Alkyl steht,
gegebenenfalls in Gegenwart eines basischen Katalysators und gegebenenfalls in Gegenwart eines Verdünnungsmittels umsetzt, oder daß man

d) für den Fall, daß in Formel (I) $R^6$ für die Gruppierung

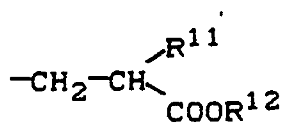

steht, worin $R^{11}$ für Halogen steht und $R^{12}$ für Alkyl steht sowie $R^1$, $R^2$, $R^3$, $R^4$ und $R^5$ die oben angegebenen Bedeutungen haben.
Verbindungen der allgemeinen Formel (I), in welcher $R^6$ für Amino steht und $R^1$, $R^2$, $R^3$, $R^4$ und $R^5$ die oben angegebenen Bedeutungen haben,
mit Natriumnitrit oder Kaliumnitrit und mit einem Hydrogenhalogenid ($HX^3$) in Gegenwart von Wasser und gegebenenfalls in Gegenwart eines organischen Lösungsmittels umsetzt und die hierbei gebildeten Diazoniumsalze der allgemeinen Formel (VII)

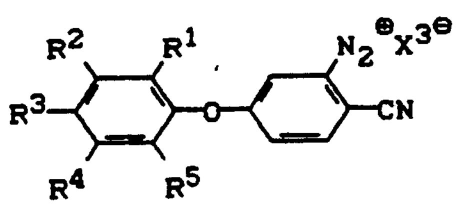

(VII)

in welcher
$R^1$, $R^2$, $R^3$, $R^4$ und $R^5$ die oben angegebenen Bedeutungen haben und
$X^3$ für Halogen steht,
mit Acrylsäureestern der Formel (VI)
$CH_2=CH-COOR^{12}$ (VI)
in welcher
$R^{12}$ für Alkyl steht,
in Gegenwart von Hydrogenhalogeniden ($HX^3$), gegebenenfalls in Gegenwart von Katalysatoren und gegebenenfalls in Gegenwart von Wasser und dem gegebenenfalls bei der Erzeugung der Verbindungen der Formel (VII) verwendeten organischen Lösungsmittel umsetzt, oder daß man

(e) für den Fall, daß in Formel (I) $R^6$ für die Gruppierung

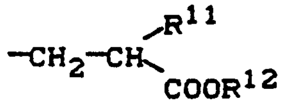

steht, worin $R^{11}$ für Wasserstoff steht und $R^{12}$ für Alkyl steht sowie $R^1$, $R^2$, $R^3$, $R^4$, und $R^5$ die oben angegebenen Bedeutungen haben,
Verbindungen der allgemeinen Formel (I), in welcher
$R^6$ für die Gruppierung

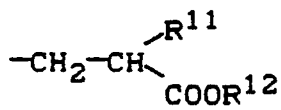

steht, worin $R^{11}$ für Halogen steht und $R^{12}$ für Alkyl steht sowie $R^1$, $R^2$, $R^3$, $R^4$ und $R^5$ die oben angegebenen Bedeutungen haben,
mit einem Dehalogenierungsmittel, gegebenenfalls in Gegenwart eines Katalysators und gegebenenfalls in Gegenwart eines Verdünnungsmittels, umsetzt.

5. Herbizide und pflanzenwuchsregulierende Mittel, gekennzeichnet durch einen Gehalt an mindestens einem substituierten Phenoxybenzonitril-Derivat der Formel (I) gemäß den Ansprüchen 1 bis 4.

6. Verfahren zur Bekämpfung von Unkräutern und zur Regulierung des Pflanzenwachstums, dadurch gekennzeichnet, daß man substituierte Phenoxybenzonitril-Derivate der Formel (I) gemäß den Ansprüchen 1 bis 4 auf die Unkräuter bzw. Pflanzen und/oder ihren Lebensraum einwirken läßt.

7. Verwendung von substituierten Phenoxybenzonitril-Derivaten der Formel (I) gemäß den Ansprüchen 1 bis 4 zur Bekämpfung von Unkräutern und/oder als Pflanzenwachstumsregulatoren.

8. Verfahren zur Herstellung von herbiziden und/oder pflanzenwuchsregulierenden Mitteln, dadurch gekennzeichnet, daß man substituierte Phenoxybenzonitril-Derivate der Formel (I) gemäß den Ansprüchen 1 bis 4 mit Streckmitteln und/oder oberflächenaktiven Substanzen vermischt.

Europäisches Patentamt

## EUROPÄISCHER RECHERCHENBERICHT

Nummer der Anmeldung

EP 90100701.3

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| X | EP - A2 - 0 034 402 (ICI) * Ansprüche * -- | 1,4 | C 07 C 255/54<br>C 07 C 255/59<br>C 07 C 253/00<br>A 01 N 37/34<br>A 01 N 37/38 |
| A | US - A - 4 526 608 (S.-F.LEE) * Ansprüche * -- | 1,5-8 | |
| A | EP - A2 - 0 166 186 (BAYER) * Ansprüche * ---- | 1,4-8 | |
| | | | **RECHERCHIERTE SACHGEBIETE (Int Cl.⁵)** |
| | | | C 07 C 253/00<br>C 07 C 255/00 |

Der vorliegende Recherchenbericht wurde für alle Patentansprüche erstellt.

| Recherchenort | Abschlußdatum der Recherche | Prüfer |
|---|---|---|
| WIEN | 06-04-1990 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82